# EUROPÄISCHE PATENTSCHRIFT

(11) **EP 1 072 239 B1**
(45) Veröffentlichungstag und Bekanntmachung des Hinweises auf die Patenterteilung: **08.10.2003**
(21) Anmeldenummer: 00115004.4
(22) Anmeldetag: 21.07.2000
(51) Int. Cl.: A61F 2/52

(54) **Verfahren zur Herstellung von Brustprothesen**
A method of manufacturing breast prostheses
Procédé de fabrication d'une prothèse mammaire

(30) Priorität: 28.07.1999 DE 19935494
(43) Veröffentlichungstag der Anmeldung: 31.01.2001
(73) Patentinhaber: Amoena Medizin-Orthopädie-Technik GmbH & Co., 83064 Raubling (DE)
(72) Erfinder: Stelter, Nils, 83101 Achenmühle (DE); Stuffer, Hans, 83131 Nussdorf/Inn (DE)
(74) Vertreter: Laufhütte, Dieter, Dr.-Ing.

(56) Entgegenhaltungen:
- DE-A- 4 413 076
- DE-U- 9 201 918

## Beschreibung

Die Erfindung betrifft ein Verfahren zur Herstellung von Brustprothesen aus einem schalenförmigen Körper aus einer additionsvemetzenden Zweikomponenten-Silikonkautschuk-Masse, der in eine diesen einhüllende Kunststoffolie eingefaßt ist, bei dem der Zweikomponenten-Silikonkautschuk-Masse ein aus Hohlkugeln oder Mikrokugeln bestehender Füllstoff mit geringer Dichte beigemengt wird, die Mischung in eine Hülle aus Kunststoff-Folien eingefüllt und in einer Form unter Wärmeeinwirkung ausgehärtet wird.

Brustprothesen dieser Art sind aus der DE 92 01 918 U bekannt. Diese Brustprothesen werden nach dem üblichen Verfahren hergestellt, nach dem die beiden Komponenten des Silikonkautschuks in ihrer Viskosität so eingestellt sind, daß sie zur Füllung der bereits zu den Prothesenhüllen miteinander verschweißten Kunststoffolien zusammen mit dem Füllstoff gepumpt und durch einen Mischer geleitet werden können. Um pumpfähig zu sein, müssen die beiden Komponenten bzw. die Mischung eine verhältnismäßig geringe Viskosität aufweisen, die dazu führt, daß die leichteren Füllstoffkugeln, die eine Dichte von < 0,1 g/cm³ aufweisen, in den in die Form eingelegten beutelförmigen Kunststoffhüllen vor ihrem Aushärten aufschwimmen, so daß die gleichmäßige Verteilung des Füllstoffes in der Silikonkautschuk-Masse verloren geht und der Füllstoff aus den unteren Bereichen der Mischung nach oben wandert und sich in dem oberen Bereich der beutelförmigen Kunststoffhüllen sammelt. Weitere Probleme bei der Verarbeitung der Mischung des Zweikomponenten-Silikonkautschuks mit den Füllstoffkugeln ergeben sich insbesondere beim Pumpen dieser Mischung. Die aus den Hohlkugeln bzw. Mikrokugeln bestehenden Füllstoffe bilden eine kompressible Komponente in der Mischung, so daß sich die gesamte Mischung nicht mehr wie eine Flüssigkeit verhält, sondern infolge von Druckunterschieden beim Pumpenlauf zu unterschiedlichen Volumenausträgen führen kann. Das ist insbesondere beim Befüllen der zu den Prothesenhüllen miteinander verschweißten Kunststoffolien problematisch.

Aus der DE 44 13 076 A1, welche als nächstkommender Stand der Technik angesehen wird, sind unterschiedliche Vorschläge bekannt, die das Problem des unerwünschten Aufschwimmens der Füllkugeln bzw. Mikrokugeln verhindern sollen. Diese Vorschläge führen aber nicht zur Abhilfe bei den Problemen der Bearbeitung der Mischung aus den Hohlkugeln bzw. Mikrokugeln mit der noch nicht vernetzten Zweikomponenten-Silikonkautschuk-Masse.

Aufgabe der Erfindung ist es daher, ein gattungsgemäßes Verfahren derart weiterzubilden, daß bei gleichmäßiger Verteilung des Füllstoffs im Prothesenkörper eine einfachere und zuverlässigere Verarbeitbarkeit der eingesetzten Stoffe während des Herstellungsverfahrens ermöglicht wird.

Erfindungsgemäß wird diese Aufgabe ausgehend von gattungsgemäßen Verfahren durch die zusätzlichen Merkmale des kennzeichnenden Teils des Anspruchs 1 gelöst. Nach dieser erfindungsgemäßen Lösung werden die Hohlkugeln oder die Mikrokugeln plastisch verformt bevor sie mit zumindest einer der beiden Komponenten der Silikonkautschuk-Masse vermischt werden.

Gemäß einer vorteilhaften Ausgestaltung der Erfindung wird das Volumen der Hohlkugeln bzw. Mikrokugeln durch Verformung unter Druck oder durch mechanische Beanspruchung reduziert. Idealerweise wird das Volumen um ca. 1/3 des Ausgangsvolumens verringert. Die ideale Volumenreduzierung hängt aber von den Eigenschaften der eingesetzten Mikrokugeln bzw. Hohlkugeln ab.

Durch die Verdichtung dieser Mikrokugeln bzw. Hohlkugeln wird deren Kompressibilität beinahe vollständig beseitigt, so daß sich das Gemisch aus der Zweikomponenten-Silikonkautschuk-Masse mit den Hohlkugeln bzw. Mikrokugeln beinahe wie eine Flüssigkeit verarbeiten läßt. Insbesondere die bei dem Pumpenanlauf auftretenden Druckunterschiede führen nicht mehr zu den unterschiedlichen Volumenausträgen, die gerade beim Befüllen der Prothesenhülle immer wieder zu Dosierungsproblemen geführt haben. Insgesamt läßt sich das Handling der Mischung erheblich verbessern. Ein besonders vorteilhafter Effekt besteht auch darin, daß die Aufschwimmtendenz der Hohlkugeln bzw. Mikrokugeln erheblich reduziert werden kann.

Zur Herstellung dieser erfindungsgemäßen Leichtprothesen wird der Füllstoff, der aus den Hohlkugeln und Mikrokugeln besteht, einer oder vorzugsweise beiden Komponenten der Silikonkautschuk-Masse zugesetzt und in die Komponenten eingerührt. Eine gleichmäßige Mischung wird durch einen Mischprozeß in einem Statik- oder Dynamik-Mischer erreicht, wobei die zuvor beispielsweise in einem Mischer mechanisch verformten Hohlkugeln bzw. Mikrokugeln sich ohne Entmischungstendenz gut in die beiden Komponenten der Silikonkautschuk-Masse einmischen lassen. Anschließend wird diese Mischung in die aus zwei verschweißten Folien bestehende vorbereitete Kunststoffhülle eingefüllt. Die derart gefüllte Kunststoffhülle wird dann in der üblichen Weise in eine Form eingelegt und in dieser unter Wärmezuführung ausgehärtet. Während des Aushärtungsprozesses unter Wärme dehnen sich die Hohlkugeln bzw. Mikrokugeln unter Volumenzunahme wieder aus. Aufgrund der Wärmeeinwirkung wird einerseits die Kunststoffhülle weich und plastisch verformbar, andererseits dehnt sich das Gas innerhalb der Hohlkugeln bzw. Mikrokugeln aus und führt dazu, daß ihre zusammengedrückte Kunststoffhülle die ursprüngliche Kugelform annimmt. Die nun wieder expandierten Hohlkugeln bzw. Mikrokugeln können aufgrund des bereits vorangeschrittenen Vernetzungszustandes in der dadurch zwischenzeitlich erhöhten Viskosität innerhalb der Zweikomponenten-Silikonkautschuk-Masse nicht mehr aufschwimmen. Vielmehr werden sie in die sich vemetzende Silikon-Masse eingebunden. Die Einfüllöffnung in dem Hüllenrand kann nach dem Befüllen oder während des Aushärtens in an sich bekannter Weise verschweißt werden.

## Patentansprüche

1. Verfahren zur Herstellung von Brustprothesen aus einem schalenförmigen Körper aus einer additionsvernetzenden Zweikomponenten-Silikonkautschuk-Masse, der in eine diesen einhüllende Kunststoffolie eingefaßt ist, bei dem der Zweikomponenten-Silikonkautschuk-Masse eine aus Hohlkugeln oder Mikrokugeln bestehender Füllstoff mit geringer Dichte beigemengt wird, die Mischung in eine Hülle aus Kunststoff-Folien eingefüllt und in einer Form unter Wärmeeinwirkung ausgehärtet wird,
**dadurch gekennzeichnet,**
**daß** die Hohlkugeln oder die Mikrokugeln plastisch verformt werden, bevor sie mit zumindest einer der beiden Komponenten der Silikonkautschuk-Masse vermischt werden.

2. Verfahren nach Anspruch 1, **dadurch gekennzeichnet, daß** das Volumen der Hohlkugeln oder Mikrokugeln durch Verformung unter Druck oder durch mechanische Beanspruchung reduziert wird.

3. Verfahren nach Anspruch 1 oder 2, **dadurch gekennzeichnet, daß** das Volumen der Hohlkugeln oder Mikrokugeln um bis ca. 1/3 des Ausgangsvolumens reduziert wird.

## Claims

1. Method of manufacturing breast prostheses from a shell-shaped body of an addition-crosslinking two-component silicone rubber composition, which is enclosed in a plastic film eveloping it, in which method a low-density filler comprising hollow beads or microbeads is admixed with the two-component silicone rubber composition, the mixture is introduced into an envelope comprising plastic films and is cured in a mould under the effect of heat,
**characterized**
**in that** the hollow beads or the microbeads are plastically deformed before they are mixed with at least one of the two components of the silicone rubber composition.

2. Method according to Claim 1, **characterized in that** the volume of the hollow beads or microbeads is reduced by deforming under pressure or by mechanical loading.

3. Method according to Claim 1 or 2, **characterized in that** the volume of the hollow beads or microbeads is reduced by up to about 1/3 of the initial volume.

## Revendications

1. Procédé de fabrication de prothèses mammaires en un corps en forme de coque à partir d'une masse de caoutchouc silicone à deux composants réticulant par addition, qui est inséré dans une feuille de matière synthétique enveloppant celui-ci, où est ajoutée à la masse de caoutchouc silicone à deux composants une matière de remplissage d'une faible densité constituée de billes creuses ou de micro-billes, le mélange est introduit dans une enveloppe en feuilles de matière synthétique et est durci dans un moule sous l'effet de la chaleur,
**caractérisé en ce que**
les billes creuses ou les micro-billes sont déformées plastiquement avant d'être mélangées avec au moins l'un des deux composants de la masse de caoutchouc silicone.

2. Procédé selon la revendication 1, **caractérisé en ce que** le volume des billes creuses ou des micro-billes est réduit par déformation sous pression ou par sollicitation mécanique.

3. Procédé selon la revendication 1 ou 2, **caractérisé en ce que** le volume des billes creuses ou des micro-billes est réduit jusqu'à environ 1/3 du volume initial.
